(19) Europäisches Patentamt
European Patent Office
Office européen des brevets

(11) **EP 1 167 950 A1**

(12) **DEMANDE DE BREVET EUROPEEN**

(43) Date de publication:
**02.01.2002 Bulletin 2002/01**

(51) Int Cl.7: **G01N 21/57**, G01N 21/21, A61B 5/103

(21) Numéro de dépôt: **01401472.4**

(22) Date de dépôt: **07.06.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **23.06.2000 FR 0008093**

(71) Demandeur: **L'OREAL**
**75008 Paris (FR)**

(72) Inventeur: **Dauga, Christophe**
**92300 Levallois-Perret (FR)**

(74) Mandataire: **Casalonga, Axel**
**BUREAU D.A. CASALONGA - JOSSE**
**Morassistrasse 8**
**80469 München (DE)**

(54) **Appareil et procédé d'examen d'une surface**

(57) L'appareil est destiné à l'examen d'une surface 9 et comprend un élément analyseur de polarisation 14 disposé sur le trajet d'un faisceau lumineux réfléchi 17 par ladite surface 9, un moyen de prise d'images numériques 13 disposé sur le trajet du faisceau réfléchi 17 par ladite surface 9 en aval de l'élément analyseur de polarisation 14, et une unité de traitement 15 apte à calculer la brillance et l'intensité d'une pluralité de pixels d'au moins une image.

FIG_2

EP 1 167 950 A1

**Description**

L'invention est relative à un appareil et à un procédé destiné à permettre l'évaluation de caractéristiques d'une surface, en particulier de la brillance, par exemple de la peau ou plus généralement de toutes surfaces kératiniques.

L'appareil est du genre comprenant une source de lumière dirigée vers la surface à examiner, d'un moyen photodétecteur sensible à la lumière renvoyée vers la surface, des moyens pour apprécier la réflexion spéculaire et la réflexion diffuse de la surface et des moyens pour évaluer la brillance à partir de l'appréciation de la réflexion spéculaire et de la réflexion diffuse. On peut consulter à ce sujet le document FR-A-2 650 890. Les essais effectués ont montré qu'un tel appareil, tout en donnant des résultats satisfaisants, avait une sensibilité et un pouvoir discriminateur relativement réduits.

On connaît également par le document EP B 0 475 803, un appareil destiné à l'examen d'une surface, comprenant une source de lumière propre à envoyer un faisceau incident sur la surface à examiner, des moyens comprenant un polariseur et au moins un analyseur permettant d'apprécier la réflexion soit avec une orientation parallèle des directions du polariseur et de l'analyseur, soit avec une orientation à angle droit, le polariseur étant disposé entre la source de lumière et la surface, tandis que l'analyseur est disposé sur le trajet du faisceau réfléchi, des moyens photodétecteurs sensibles à la lumière renvoyée par la surface étant en outre prévus. La source de lumière est directive et le faisceau incident polarisé tombe sur la surface à étudier selon un angle d'incidence compris entre 0 et 90°, limites exclues, la direction de polarisation du faisceau incident étant perpendiculaire au plan d'incidence. L'appareil est agencé pour mesurer la réflexion suivant au moins deux directions de réflexion différentes, l'une sensiblement symétrique de la direction incidente relativement à la normale à la surface. L'appareil comporte des moyens pemettant d'effectuer, pour chaque direction de réflexion, la différence entre la réflexion dans des directions de polarisation et d'analyse parallèles et la réflexion avec les directions de polarisation et d'analyse perpendiculaires, les différences ainsi obtenues constituant une appréciation de la brillance dite spéculaire et de la brillance dite diffuse.

Un tel appareil fonctionne convenablement, mais ne permet l'examen en un instant donné que d'une surface élémentaire ou d'un point.

L'invention propose de fournir des données de brillance relatives à l'ensemble des points d'une surface à un instant donné.

L'invention propose de fournir un appareil d'examen de surface amélioré.

L'appareil, selon un aspect de l'invention, est destiné à l'examen d'une surface et comprend un élément analyseur de polarisation disposé sur le trajet d'un faisceau lumineux réfléchi par ladite surface. L'appareil comprend, en outre, un moyen de prise d'images numériques disposé sur le trajet du faisceau réfléchi par ladite surface en aval de l'élément analyseur de polarisation, et une unité de traitement apte à calculer la brillance et l'intensité d'une pluralité de points de ladite surface à partir des pixels d'au moins deux images de ladite surface.

L'examen peut s'effectuer à distance de la peau. On évite ainsi le risque de modifier les caractéristiques que l'on cherche à mesurer.

Lesdites deux images seront prises pour des polarisations différentes.

De préférence, l'appareil comprend une source de lumière polarisée apte à envoyer un faisceau incident sur ladite surface à examiner.

De préférence, la lumière issue de ladite source est sensiblement isotrope.

Dans un mode de réalisation de l'invention, la lumière issue de ladite source est sensiblement blanche.

Dans un mode de réalisation de l'invention, le spectre de la lumière issue de ladite source est sensiblement égal au spectre solaire.

Dans un mode de réalisation de l'invention, l'élément analyseur de polarisation comprend un moyen de transmission de la polarisation croisée et un moyen de transmission de la polarisation parallèle, lesdits moyens de transmission étant actifs alternativement.

Dans un mode de réalisation de l'invention, l'élément analyseur de polarisation est rotatif.

Dans un autre mode de réalisation de l'invention, l'élément analyseur de polarisation comprend un moyen de commutation électrique.

Le moyen de prise d'images numériques peut être sensible à la couleur.

L'unité de traitement comprendra, avantageusement, un microprocesseur, des moyens mémoires et un logiciel stocké dans les moyens mémoires.

L'invention concerne également un procédé d'examen d'une surface, dans lequel on effectue une analyse de la polarisation d'un faisceau lumineux réfléchi par ladite surface, on prend des images numériques de polarisations particulières dudit faisceau réfléchi, et on calcule la brillance et l'intensité d'une pluralité de points de l'image à partir des pixels d'au moins deux images de ladite surface.

Dans un mode de réalisation de l'invention, ladite surface est gauche.

Dans un mode de réalisation de l'invention, on prend des images numériques monochromes.

Dans un mode de réalisation de l'invention, on prend des images numériques multichromes.

L'invention concerne également un programme d'ordinateur comprenant des moyens de code programme pour mettre en oeuvre des étapes de déploiement du dispositif, lorsque ledit programme fonctionne sur un ordinateur.

L'invention concerne également un support capable d'être lu par un dispositif de lecture de moyens de code programme qui s'y trouvent stockés et qui sont aptes à la mise en oeuvre des étapes de déploiement du dispositif, lorsque ledit programme fonctionne sur un ordinateur.

On entend ici par point une partie élémentaire de ladite surface à examiner, de dimensions correspondant à un pixel de l'image obtenue par le moyen de prises d'images.

En d'autres termes, on éclaire la surface à examiner qui peut être un ongle ou une partie d'un ongle, le visage ou une partie du visage, etc., d'une personne. L'éclairage est effectué à partir d'une source de lumière ou d'une pluralité de sources de lumière, de façon que ledit éclairage soit le plus isotrope possible. On polarise la lumière issue du moyen d'éclairage, par exemple au moyen d'un polariseur fixe. On analyse la polarisation de la lumière réfléchie par la surface à examiner, de façon qu'on sépare la partie de la lumière dont la polarisation a été conservée et la partie de la lumière dont la polarisation a changé, et ce pour toute la surface à examiner.

On prend des images numériques en aval de l'analyseur de polarisation, par exemple au moyen d'une caméra matricielle, pour calculer le degré de polarisation de chaque pixel de l'image. On en déduit par un traitement numérique une information relative à la brillance de l'image. A cet effet, on prendra au moins deux images, de préférence trois images, en particulier dans le cas d'un analyseur de polarisation de type rotatif et d'une surface gauche. L'examen se fait sans contact, pour accroître le confort de la personne dont une surface est examinée, supprimer le risque d'imprécision ou d'erreur lié à une modification de la forme, concave ou convexe, de la surface en raison du contact, et supprimer le risque de modification de la brillance et donc d'erreur de mesure, en particulier dans le cas d'une surface ayant reçu au préalable un produit de traitement, du genre maquillage, coloration ou soin, cas auquel un contact est susceptible de modifier la répartition surfacique.

La présente invention sera mieux comprise et d'autres avantages apparaîtront à la lecture de la description détaillée de quelques modes de réalisation pris à titre d'exemples nullement limitatifs et illustrés par les dessins annexés, sur lesquels :

- la figure 1 est une vue schématique de la réflexion de deux rayons lumineux;
- la figure 2 est une vue schématique d'un appareil selon un mode de réalisation de l'invention;
- la figure 3 est une courbe montrant l'évolution de l'intensité d'un pixel en fonction de l'angle de l'analyseur; et
- la figure 4 est un organigramme d'étapes de procédé.

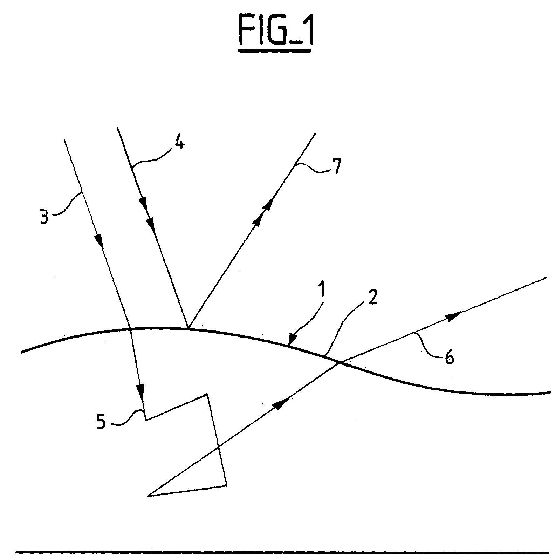

Sur la figure 1, est illustré un objet 1 pourvu d'une surface 2 que l'on éclaire par une lumière dont deux rayons lumineux incidents 3 et 4 sont représentés. Le rayon lumineux 3 traverse la surface 2 et pénètre dans l'objet 1 en suivant une trajectoire 5, puis ressort sous la forme d'un rayon réfléchi diffusé 6. Cette réflexion diffuse ou "couleur" correspond à une lumière qui pénètre dans l'objet, se réfléchit à l'intérieur de celui-ci puis est réémise à l'extérieur. Les caractéristiques du rayon réfléchi 6 dépendent de l'objet 1. Le rayon lumineux incident 4 se réfléchit sur la surface 2 sous la forme d'un rayon réfléchi 7. Ce type de réflexion est dit spéculaire et est également appelé brillance. La lumière due à la brillance possède les caractéristiques spectrales de la lumière incidente. La forme du diagramme de luminance du rayon réfléchi 7 dépend de la rugosité de la surface 2.

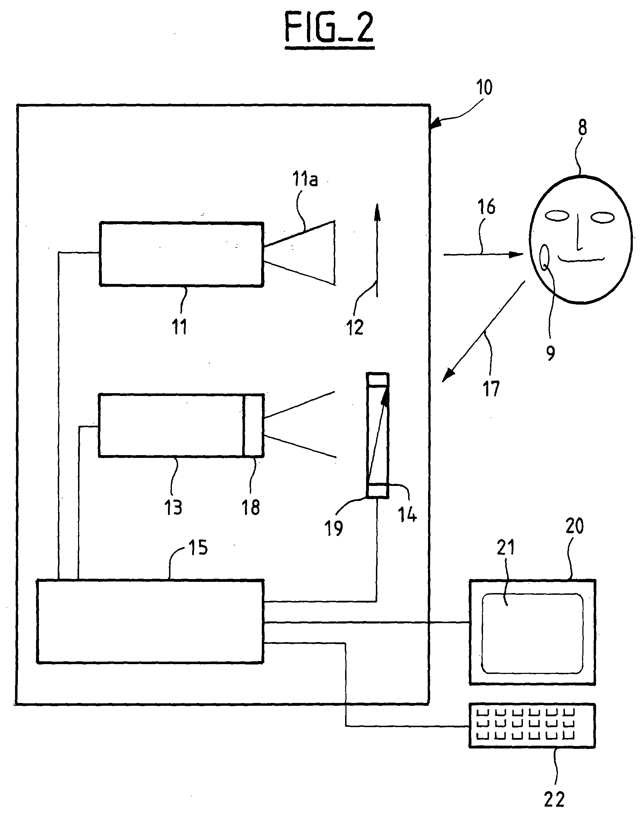

Comme on peut le voir sur la figure 2, on souhaite examiner le visage 8 d'une personne, plus particulièrement une surface 9 du visage 8. A cette fin, on prévoit un appareil d'examen 10 comprenant une source de lumière 11, un polariseur 12 de type fixe, une caméra matricielle 13, un analyseur de polarisation 14 et une unité de traitement 15.

La source de lumière 11 est disposée de façon qu'elle éclaire la surface 9. La lumière émise sera la plus isotrope possible car il s'avère que la mesure peut être sensible à l'incidence des rayons lumineux sur la surface 9. La source de lumière 11 devra, en tout état de cause, reproduire le mieux possible le spectre solaire, c'est-à-dire émettre une lumière blanche.

Plus particulièrement, la source de lumière 11 comprend une lampe flash ou continue à spectre étendu, du genre Xénon ou tube fluorescent, ou des diodes électroluminescentes multicolores. La source de lumière 11 comprend également un système optique 11a du type réflecteur, miroir, objectif, condenseur de lumière et fibres optiques, pour diriger la lumière dans un angle déterminé et adapté vers la surface 9.

Le polariseur fixe 12 est disposé sur le trajet du faisceau lumineux incident 16 émis par la source de lumière 11, en d'autres termes entre la source de lumière 11 et la surface 9. En aval du polariseur fixe 12, dans le sens de propagation du faisceau lumineux incident 16, la lumière est polarisée.

La caméra matricielle 13 peut être du type CCD et est installée pour recevoir le faisceau lumineux réfléchi 17 issu de la surface 9 lorsque la source de lumière 11 est active. La caméra matricielle 13 peut être pourvue d'un objectif réglable 18.

L'analyseur de polarisation 14 est disposé sur le trajet du faisceau réfléchi 17, en d'autres termes entre la surface 9 et la caméra matricielle 13. L'orientation de l'analyseur de polarisation 14 peut se faire selon un axe parallèle à celui du faisceau réfléchi 17 entre au moins deux positions, par exemple décalées d'un angle de 90°. On peut ainsi séparer la partie du faisceau lumineux 17 issue d'une réflexion spéculaire et la partie issue d'une réflexion diffuse, étant précisé que dans l'une de ces deux positions, l'analyseur de polarisation 14 présente la même polarisation que le polariseur fixe 12. Si ce n'est pas le cas, un éventuel traitement numérique ultérieur peut permettre de retrouver les mêmes résultats. L'analyseur 14 peut être un polariseur orientable, avantageusement pourvu d'un moteur 19 apte à l'entraîner en rotation. Le moteur 19 pourra être du type pas à pas et si possible à haute résolution pour effectuer une polarisation précise.

La source de lumière 11, la caméra 13 et le moteur 19 du polariseur orientable 14, sont reliés à l'unité de traitement 15 qui est du type comprenant au moins une mémoire, au moins un microprocesseur, et au moins un programme de commande stocké dans une mémoire et apte à être exécuté par le ou les microprocesseurs. L'unité de traitement 15 est apte à commander la mise en marche et l'arrêt de la source de lumière 11, la prise d'image par la caméra 13 et, le cas échéant, le réglage de l'objectif 18, et les orientations adéquates de l'analyseur 14.

L'unité de traitement 15 peut également être reliée à des dispositifs extérieurs à l'appareil d'examen 10, par exemple par un moniteur 20 pourvu d'un écran 21 permettant la visualisation d'images pouvant représenter, soit la surface 9, soit des résultats de l'examen effectué, c'est-à-dire des données traitées par l'unité de traitement 15. L'unité de traitement 15 peut également être reliée à un clavier 22 permettant à un opérateur d'entrer des informations ou des commandes.

Le polariseur orientable peut être du genre à orientation électro-optique, par exemple "polarization rotator" de la Société DISPLAYTECH, ou à orientation mécanique, par exemple avec un moteur et une pluralité de filtres montés sur une roue entraînée par le moteur. En variante, on peut également prévoir un cube séparateur de polarisation, par exemple "Beamsplitter" de la Société ORIEL, mais qui nécessiterait toutefois l'utilisation de deux caméras de mesure. De façon préférée, l'analyseur 14 est un système électro-optique qui commute en temps réel et peut être synchronisé par voie externe reliée à l'unité de commande 15. L'analyseur 14 permet de séparer la brillance qui est une composante de lumière réfléchie de façon spéculaire par la surface 9, de la couleur qui est une composante de lumière rétrodiffusée par la surface 9 lorsqu'il est placé devant la caméra matricielle 13. Lorsque l'analyseur 14 est dans la même direction de polarisation que le faisceau lumineux incident 16, la caméra 13 capte la lumière réfléchie par la surface 9 ainsi que la moitié de la composante dépolarisée. Lorsque l'analyseur 14 est dans une direction orthogonale de polarisation du faisceau lumineux incident 16, la caméra 13 capte seulement la moitié de la composante dépolarisée. L'unité de traitement 15 effectue l'opération algébrique de soustraction pour obtenir la composante lumineuse liée à la brillance et de multiplication pour obtenir la composante lumineuse liée à la couleur.

Préférablement et pour une meilleure précision, on effectue l'acquisition d'un nombre suffisant d'images pour des positions quelconques de l'analyseur 14. Une analyse de Fourier du signal mesuré, effectuée par l'unité de traitement 15, permet de calculer le degré de polarisation du faisceau lumineux réfléchi 17 et d'en extraire la composante de brillance ainsi que la composante de couleur de la surface 9.

L'objectif 18 de la caméra 13 permet de focaliser le faisceau lumineux réfléchi sous un certain angle solide sur un élément photosensible tel qu'une matrice de cellules CCD. Pour chaque position de l'analyseur 14, une image est acquise via une carte d'acquisition d'image associée soit à l'unité de traitement 15, soit à la caméra 13, par exemple la carte "ICPCI" de la Société IMAGING TECHNOLOGY. L'acquisition d'image est réalisée lorsque l'analyseur est en position stationnaire après avoir tourné. L'acquisition de deux images en polarisations parallèle et croisée s'effectue en quelques centaines de millisecondes.

La matrice de cellules CCD assure la fonction de radiomètre. Si l'on s'intéresse à la répartition spectrale du faisceau lumineux réfléchi 17, par exemple la densité spectrale de la brillance pour la composante spéculaire et la densité spectrale de la couleur pour la composante rétrodiffusée, on pourra utiliser un spectromètre. La fonction de radiomètre et la fonction de spectromètre peuvent être combinées au sein d'un même appareil tel qu'un spectroradiomètre.

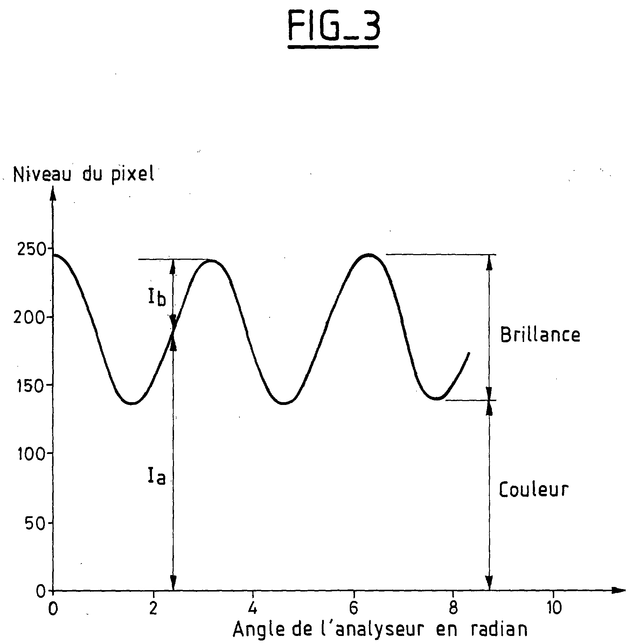

Sur la figure 3, est illustrée une courbe montrant le niveau d'un pixel en ordonnée en fonction de l'angle de l'analyseur en abscisse. En effet, si la surface à examiner est éclairée par un faisceau lumineux polarisé, le rayonnement correspondant à la brillance reste polarisé alors que celui correspondant à la couleur se dépolarise. La rotation de l'analyseur permet de déterminer la contribution de la brillance et de la couleur en chaque point de l'image. Lorsque l'on tourne l'analyseur, l'intensité en un point donné varie de manière sensiblement sinusoïdale. Lors de la réflexion sur la surface à examiner, l'orientation de la polarisation de la partie du faisceau lumineux réfléchie correspondant à la brillance tourne d'une quantité liée à l'angle entre le faisceau incident et la normale à la surface à examiner en ce point.

Si la surface à examiner est plane, l'angle dont la polarisation tourne est le même en chaque point. Il suffit alors de deux prises d'image selon deux angles différents de l'analyseur, l'un correspondant au maximum, l'autre au minimum de la courbe de la figure 3, pour déterminer la part de la couleur et la part de la brillance en chaque point de l'image. Les positions angulaires de l'analyseur peuvent être déterminées de façon automatique facilement car elles correspondent à un maximum et à un minimum général de l'image.

Si la surface à examiner est gauche, il apparaît un déphasage en chaque point de l'image, et il faut utiliser au moins trois positions différentes de l'analyseur.

L'intensité en chaque point peut s'écrire

$$I = I_a + I_b \cos(2\theta)$$

où $\theta$ est l'angle entre l'analyseur et la verticale, $I_a$ est la valeur moyenne du signal I, et $I_b$ est la demi différence entre le maximum et le minimum du signal I.

Si, par exemple on utilise trois positions, régulièrement espacées de 45°, on obtient en chaque point de l'image :

$$I_o = I_a + I_b \cos(2\theta_o)$$

$$I_{45} = I_a + I_b \cos(2(\theta_o + \tfrac{\pi}{4})) = I_a + I_b \cos(2\theta_o + \tfrac{\pi}{2}) = I_a - I_b \sin(2\theta_o)$$

$$I_{90} = I_a + I_b \cos(2(\theta_o + \tfrac{\pi}{2})) = I_a + I_b \cos(2\theta_o + \pi) = I_a - I_b \cos(2\theta_o)$$

soit :

$$I_a = \frac{I_o + I_{90}}{2}$$

$$I_b = [(I_{90} - I_a)^2 + (I_{45} - I_a)^2]^{1/2} = \frac{1}{2}[(I_{90} - I_0)^2 + (I_{45} - I_o - I_{90})^2]^{1/2}$$

or

$$I_{brillance} = 2I_b \text{ et } I_{couleur} = 2(I_a - I_b) ;$$

on a donc :

$$I_{brillance} = [(I_{90} - I_o)^2 + (I_{45} - I_o - I_{90})^2]^{1/2}$$

$$I_{couleur} = I_o + I_{90} - [(I_{90} - I_o)^2 + (I_{45} - I_o - I_{90})^2]^{1/2}$$

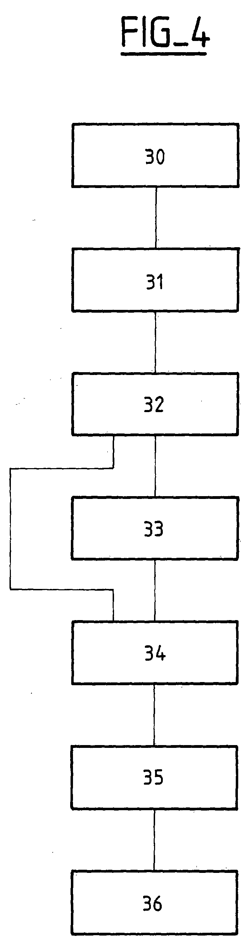

Les différentes étapes du procédé d'examen de la surface 9 sont illustrées sur la figure 4.

A l'étape 30, l'opérateur, voire même l'utilisateur, commande le début de l'examen, par exemple au moyen du clavier 22.

A l'étape 31, l'unité de traitement 15 ayant reçu la commande de démarrage envoie un ordre d'activation à la source lumineuse 11 qui se met à émettre le faisceau lumineux incident 16.

A l'étape 32, la caméra 13 effectue une prise d'image pour un angle de l'analyseur 14 de 0°.

A l'étape 33, la caméra 13 effectue une prise d'image pour un angle de l'analyseur 14 égal à 45° et à l'étape 34, la caméra 13 effectue une prise d'image pour un angle de l'analyseur 14 égal à 90°.

Dans le cas où l'opérateur estime que la surface à examiner 9 est plane, en particulier s'il s'agit d'une surface de très faible taille, l'étape 33 peut être omise.

A l'étape 35, l'unité de traitement 15 effectue le calcul numérique permettant de séparer les composantes de brillance et de couleur dans le faisceau lumineux réfléchi 17, en d'autres termes d'obtenir une image de brillance et une image de couleur.

A l'étape 36, le résultat du traitement est affiché sur l'écran 21, sous la forme qui paraîtra la mieux adaptée, courbe, graphique, diagramme, etc.

Lors des étapes 35 et 36, on prévoit également que l'analyseur 14 revienne à un angle de 0° pour être prêt à commencer l'examen d'une autre surface.

Dans un autre mode de réalisation de l'invention, l'analyseur effectue une rotation continue au cours de laquelle plusieurs images sont prises par la caméra 13. Pour une surface à examiner donnée, l'estimation de la brillance sera d'autant plus précise que le nombre d'images qui auront été prises sera élevé.

Lors de l'étape 35 de traitement par l'unité 15, on tiendra compte du fait que l'oeil humain est sensible au contraste entre la brillance et la couleur plus qu'à la brillance seule. A titre d'exemple, un noir à niveau de brillance donné semble plus brillant qu'un blanc du même niveau de brillance. L'unité 15 effectuera donc, d'une part, un calcul permettant d'obtenir une cartographie de la brillance et, d'autre part, un calcul de la brillance rapportée à la couleur. On affichera, de préférence, une information relative à la brillance rapportée à la couleur qui est la plus pertinente en matière d'impression perçue par l'oeil humain.

Ainsi, l'appareil d'examen de surface permet de mesurer la brillance et la brillance relative de tous types de surfaces, en particulier kératiniques, par exemple les cheveux, les lèvres, les ongles, la peau, etc.

Ces différentes surfaces peuvent avoir reçu au préalable différents types de produits de traitement, par exemple de soin, de coloration, de maquillage, etc. Dans le cas du maquillage, l'appareil d'examen de surface permet d'estimer l'aspect plus ou moins mat de la surface, notamment de la peau, maquillée.

**Revendications**

1. Appareil destiné à l'examen d'une surface (9), comprenant un élément analyseur de polarisation (14) disposé sur le trajet d'un faisceau lumineux réfléchi (17) par ladite surface, **caractérisé par le fait qu'**il comprend un moyen de prise d'images numériques (13) disposé sur le trajet du faisceau réfléchi par ladite surface en aval de l'élément analyseur de polarisation, et une unité de traitement (15) apte à calculer la brillance et l'intensité d'une pluralité de points de ladite surface à partir de pixels d'au moins deux images de ladite surface.

2. Appareil selon la revendication 1, **caractérisé par le fait qu'**il comprend une source de lumière polarisée apte à envoyer un faisceau incident (16) sur ladite surface à examiner.

3. Appareil selon la revendication 2, **caractérisé par le fait que** la lumière issue de ladite source est sensiblement isotrope.

4. Appareil selon la revendication 2 ou 3, **caractérisé par le fait que** la lumière issue de ladite source est sensiblement blanche.

5. Appareil selon la revendication 2 ou 3, **caractérisé par le fait que** le spectre de la lumière issue de ladite source est sensiblement égal au spectre solaire.

6. Appareil selon l'une quelconque des revendications précédentes, **caractérisé par le fait que** l'élément analyseur de polarisation comprend un moyen de transmission de la polarisation croisée et un moyen de transmission de la polarisation parallèle, lesdits moyens de transmission étant actifs alternativement.

7. Appareil selon la revendication 6, **caractérisé par le fait que** l'élément analyseur de polarisation est rotatif.

8. Appareil selon la revendication 6, **caractérisé par le fait que** l'élément analyseur de polarisation comprend un moyen de commutation électrique.

9. Procédé d'examen à distance d'une surface, dans lequel on effectue une analyse de la polarisation d'un faisceau lumineux réfléchi par ladite surface, on prend des images numériques de polarisations particulières dudit faisceau réfléchi, et on calcule la brillance et l'intensité d'une pluralité de points de ladite surface à partir de pixels d'au moins deux images de ladite surface.

10. Procédé selon la revendication 9, dans lequel ladite surface est gauche.

11. Procédé selon la revendication 9 ou 10, dans lequel on prend des images numériques monochromes.

12. Procédé selon la revendication 9 ou 10, dans lequel on prend des images numériques multichromes.

13. Programme d'ordinateur comprenant des moyens de code programme pour mettre en oeuvre les étapes du procédé selon l'une quelconque des revendications 9 à 12, lorsque ledit programme fonctionne sur un ordinateur.

14. Support capable d'être lu par un dispositif de lecture de moyens de code programme qui s'y trouvent stockés et qui sont aptes à la mise en oeuvre des étapes du procédé selon l'une quelconque des revendications 9 à 12, lorsque ledit programme fonctionne sur un ordinateur.

# FIG_1

# FIG_2

# FIG_3

# FIG_4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,Y<br><br>A | EP 0 475 803 A (OREAL)<br>18 mars 1992 (1992-03-18)<br>* colonne 2, ligne 24 - ligne 42 *<br>* colonne 2, ligne 53 - colonne 3, ligne 2 *<br>* colonne 3, ligne 36 - ligne 43 *<br>* colonne 4, ligne 21 - ligne 26 *<br>* colonne 4, ligne 39 - ligne 46 *<br>--- | 1,2,4,<br>6-8<br>9,10 | G01N21/57<br>G01N21/21<br>A61B5/103 |
| Y<br><br><br>A | WO 99 37980 A (JACQUES STEVEN L ;PROVIDENCE HEALTH SYSTEM (US))<br>29 juillet 1999 (1999-07-29)<br>* page 3, ligne 12 - ligne 18 *<br>* page 7, ligne 5 - ligne 6 *<br>* page 8, ligne 11 - ligne 13 *<br>* page 8, ligne 23 - ligne 30 *<br>--- | 1,2,4,<br>6-8<br><br>11,12 | |
| D,A | FR 2 650 890 A (OREAL)<br>15 février 1991 (1991-02-15)<br>* page 7, ligne 18 - ligne 21 *<br>* page 7, ligne 33 - page 8, ligne 1 *<br>--- | 1-4 | |
| A | US 5 557 324 A (WOLFF LAWRENCE B)<br>17 septembre 1996 (1996-09-17)<br>* le document en entier *<br>--- | 1 | DOMAINES TECHNIQUES RECHERCHES (Int.Cl.7)<br><br>G01N<br>A61B<br>G01J |
| A | US 6 032 071 A (BINDER MICHAEL)<br>29 février 2000 (2000-02-29)<br>* le document en entier *<br>--- | 1 | |
| A | EP 0 726 456 A (ASAHI CHEMICAL IND)<br>14 août 1996 (1996-08-14)<br>* page 6, ligne 27 - ligne 34 *<br>* page 6, ligne 48 - ligne 50 *<br>----- | 1 | |

**Office européen des brevets**

**RAPPORT DE RECHERCHE EUROPEENNE**

Numéro de la demande

EP 01 40 1472

Le présent rapport a été établi pour toutes les revendications

| Lieu de la recherche | Date d'achèvement de la recherche | Examinateur |
|---|---|---|
| LA HAYE | 20 septembre 2001 | Verdoodt, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 01 40 1472

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

20-09-2001

| Document brevet cité au rapport de recherche | | Date de publication | Membre(s) de la famille de brevet(s) | | Date de publication |
|---|---|---|---|---|---|
| EP  0475803 | A | 18-03-1992 | FR | 2665959 A1 | 21-02-1992 |
| | | | CA | 2049264 A1 | 17-02-1992 |
| | | | DE | 69109413 D1 | 08-06-1995 |
| | | | DE | 69109413 T2 | 08-02-1996 |
| | | | EP | 0475803 A1 | 18-03-1992 |
| | | | JP | 5220130 A | 31-08-1993 |
| | | | US | 5198875 A | 30-03-1993 |
| WO 9937980 | A | 29-07-1999 | US | 6177984 B1 | 23-01-2001 |
| | | | AU | 732108 B2 | 12-04-2001 |
| | | | AU | 2335199 A | 09-08-1999 |
| | | | EP | 1049913 A1 | 08-11-2000 |
| | | | WO | 9937980 A1 | 29-07-1999 |
| FR 2650890 | A | 15-02-1991 | FR | 2650890 A1 | 15-02-1991 |
| US 5557324 | A | 17-09-1996 | CA | 2146994 A1 | 11-05-1994 |
| | | | EP | 0729687 A1 | 04-09-1996 |
| | | | JP | 8503313 T | 09-04-1996 |
| | | | WO | 9410795 A1 | 11-05-1994 |
| US 6032071 | A | 29-02-2000 | AT | 403654 B | 27-04-1998 |
| | | | AT | 223394 A | 15-09-1997 |
| | | | WO | 9616698 A2 | 06-06-1996 |
| | | | AU | 707671 B2 | 15-07-1999 |
| | | | AU | 3897595 A | 19-06-1996 |
| | | | CA | 2206273 A1 | 06-06-1996 |
| | | | EP | 0794731 A2 | 17-09-1997 |
| EP 0726456 | A | 14-08-1996 | JP | 6222002 A | 12-08-1994 |
| | | | EP | 0726456 A1 | 14-08-1996 |
| | | | FI | 961769 A | 19-06-1996 |
| | | | KR | 169892 B1 | 30-03-1999 |
| | | | US | 5974160 A | 26-10-1999 |
| | | | CA | 2175071 A1 | 04-05-1995 |
| | | | CN | 1133633 A | 16-10-1996 |
| | | | WO | 9512120 A1 | 04-05-1995 |

EPO FORM P0460

Pour tout renseignement concernant cette annexe : voir Journal Officiel de l'Office européen des brevets, No. 12/82